# EUROPEAN PATENT SPECIFICATION

(11) **EP 2 184 019 B1**
(45) Date of publication and mention of the grant of the patent: **09.12.2015**
(21) Application number: 08791639.1
(22) Date of filing: 25.07.2008
(51) Int. Cl.: A61B 17/12, A61B 17/00

(54) **DEVICE FOR OPENING/CLOSING BIOLOGICAL TISSUE**
VORRICHTUNG ZUM ÖFFNEN/VERSCHLIESSEN VON BIOLOGISCHEM GEWEBE
DISPOSITIF D'OUVERTURE/DE FERMETURE DE TISSU BIOLOGIQUE

(30) Priority: 28.08.2007 JP 2007221898; 28.08.2007 JP 2007221908
(43) Date of publication of application: 12.05.2010
(73) Proprietor: Terumo Kabushiki Kaisha, Tokyo 151-0072 (JP)
(72) Inventor: KATOU, Yukitoshi, Ashigarakami-gun Kanagawa 259-0151 (JP); KURODA, Yasuyuki, Ashigarakami-gun Kanagawa 259-0151 (JP)
(74) Representative: Dossmann, Gérard
(86) International application number: PCT/JP2008/063395
(87) International publication number: WO 2009/028285

(56) References cited:
- WO-A2-99/12489
- WO-A2-2004/086944
- WO-A2-2004/087235
- WO-A2-2007/030433
- JP-A- 2005 525 843
- JP-A- 2006 521 186
- JP-A- 2007 098 139
- JP-A- 2007 519 489
- US-A1- 2006 271 089
- US-A1- 2007 123 851
- US-A1- 2007 150 000

## Description

### TECHNICAL FIELD

The present invention relates to a biological tissue closing device which closes a defect occurring in a living body.

### BACKGROUND ART

Recently, a patent foramen ovale (hereinafter, referred as to PFO: Patent Foramen Ovale) has been cited for a cardiogenic factor of a stroke and a hemi headache. A PFO is a symptom in which a foramen ovale which makes blood shunt for the right to the left in the heart of a fetal period remains even if a fetus becomes adult and it is said that 20 to 30% of adults possess it.

The foramen ovale occurs at a septum secundum (Septum Secundum, hereinafter, referred to as atrial septum secundum) of the heart and the pressure on the left atrium exceeds the pressure on the right atrium side in the heart on a normal occasion, so that it is occluded by a septum primum (Septum Primum, hereinafter, referred to as foramen ovale valve), but when the pressure on the right atrium side exceeds the pressure on the left atrium side on a strain occasion (for example, when coughing, when holding on) or the like, the foramen ovale valve opens to the left atrium side and it happens that blood flows from the right atrium side (venous side) into the left atrium side (arterial side). When a thrombus is included in this blood, it happens that the thrombus is shifted from the venous side to the arterial side, flows in a route of left atrium - left ventricle -aorta -brain, and it becomes a factor for a stroke, hemi headache or the like.

For a treatment with respect to such a disease, a treatment by a percutaneous catheter procedure is considered to be a desirable method if the same effect as an open heart surgery can be obtained.

A device of a closing technique using the percutaneous catheter can be used also in case of closing defects such as a congenital atrial septum secundum defect (ASD), a PFO, a ventricular septal defect (VSD) and a patent ductus arteriosus (PDA), and the device in the past is a device sandwiching the foramen ovale valve and the atrial septum secundum by using disk-shaped membranes or anchor members for closing the defect and these are indwelled in the body.

The membranes mentioned above or anchor members are foreign substances for a human body and moreover, a thrombus is easy to be attached. In particular, when a thrombus is attached on a disk-shaped membrane or the like on the left atrium side, this flows and there is a possibility that it becomes a cause of a stroke, and there is also a fear that a foramen ovale valve of a thin wall thickness is broken. In addition, these members are not position-fixed in a state of being sandwiched and there is also a possibility of causing a positional displacement.

Consequently, recently, there has been proposed a PFO closing device which is described in a Patent Document 1 of WO2004/086944 A2 (see summary, FIG. 10 and the like). This PFO closing device is a device in which an apparatus is inserted into the foramen ovale from the right atrium toward the left atrium, a foramen ovale valve is pulled to the foramen ovale so as to close it and the tissue is to be fused by applying electric energy. However, the foramen ovale, the foramen ovale valve and the atrial septum secundum are different not only in small/large sizes but also in a condition of thicknesses, shapes or the like depending on persons and according to circumstances, the size or the like of the apparatus is restricted a lot. Also, even on an occasion when the procedure is performed, there is a fear that it becomes difficult to pull various forms of foramen ovale valves to the foramen ovale at anytime and certainly.

Consequently, as shown in a Patent Document 2 of a Japanese Patent Application No. 2006-47636 (see summary, FIG. 10 and the like), the present patent applicant proposed previously a PFO closing device in which the foramen ovale valve and the atrial septum secundum are sandwiched by a pair of electrodes and the tissue is fused by applying electric energy from both the electrodes. This PFO closing device uses clamping means in which a stick portion and a sandwich member are composed of a pair of electrodes and with respect to one of them, the stick portion composed of a needle electrode is stuck into the foramen ovale valve and thereafter, the foramen ovale valve and the atrial septum secundum are sandwiched with respect to the sandwich member which is the other electrode, the biological tissue is applied with electric energy, and the fusion is carried out. If this PFO closing device is used, foreign substances are not indwelled in the body, the constitution becomes simple, also the procedure becomes easy and the foramen ovale valve and the atrial septum secundum can be fused certainly.

However, there is provided in this device with a guiding catheter on the outside of a fine catheter and the fine catheter is used by being stored in the guiding catheter and moreover, for the sake of attempting simplification of the operation, the stick portion which easily injures the blood vessel is provided position-fixedly at the distal tip of the fine catheter and there is employed a constitution in which only the sandwich member moves with respect to the fine catheter, so that the sticking by the stick portion must be executed by moving the fine catheter in the guiding catheter and therefore, there is a problem that the degree of freedom for selecting the sticking position is not enough.

In particular, the stick operation is performed in a state in which the stick portion is protruded from the distal tip of the guiding catheter and by sticking the foramen ovale valve while moving the whole catheter forward and backward, so that the sticking position will be on an extension line of the catheter axial line and it is possible to execute the stick at a desired position by using a positioning member, but when moving the stick portion, there is a fear that miss sticking into the front side of the foramen ovale valve will occur caused by a body movement which happens in case of a shallow depth of anesthesia. It is ideal to select the sticking position to be right under the position at which the foramen ovale valve and the atrial septum secundum are overlapped, but it is extremely difficult to adjust manually the position of the needle member of the stick portion, which is positioned at the distal tip of the fine long catheter.
Patent Document 1: WO2004/086944 A2 (see the abstract, FIG. 10 etc.)
Patent Document 2: Japanese patent application No. 2006-47636 (Abstract, FIG. 10 etc.) Document US 2006/271089 discloses a device for closing a biological tissue according to the preamble of claim 1.

### DISCLOSURE OF THE INVENTION

### PROBLEM TO BE SOLVED BY THE INVENTION

The present invention is invented to solve the problem mentioned above and has an object to provide a biological tissue closing device in which, by forming the stick portion and the sandwich member movable independently with respect to the catheter, the degree of freedom for selecting the sticking position is heightened, it becomes possible to select a more ideal sticking position, and an improvement in smoothness, safety and also certainty of the procedure is achieved.

### MEANS FOR SOLVING THE PROBLEM

The biological tissue closing device according to the present invention which achieves the aforementioned object is a device making a stick portion of clamping means and a sandwich member movable respectively independently with respect to the catheter and in particular, is characterized in that the stick portion mentioned above is protruded in the side direction of the catheter.

### EFFECT OF THE INVENTION

According to the present invention, by making a stick portion and a sandwich member of clamping means movable independently with respect to a catheter respectively in the biological tissue closing device, it is possible, with respect to aforesaid stick portion, to select and stick an arbitrary position, for example, such an ideal sticking position as a directly-close position of a position at which a foramen ovale valve and an atrial septum secundum which are biological tissues are overlapped and thus, it is possible to achieve an improvement in smoothness, safety and also certainty of the procedure.

In particular, if aforesaid stick portion is to be retracted from the distal region of aforesaid catheter in a tilting manner with respect to the axial line of aforesaid catheter, it is possible to protrude aforesaid stick portion toward the side direction of the catheter and when the catheter is moved in the axial direction and comes to a predetermined position, it is possible to execute the stick and it is possible to execute the stick at an ideal sticking position easily.

If aforesaid stick portion is constituted by a needle member and with respect to this needle member, if deforming aforesaid needle member elastically and protruding it into a lumen depending on the lumen shape of a holder provided at a distal portion of aforesaid catheter, protrusion in the side direction of aforesaid needle member becomes possible without requesting another means. The device constitution is also simplified and it becomes profitable also with respect to the cost.

By employing a constitution in which there is provided on the distal side of the stick portion a plurality of needle members which are mutually expandable and contractible, the stick needle contacts the foramen ovale valve in a wide region by the plurality of the needle members and it is possible to sandwich the foramen ovale valve and the atrial septum secundum after the sticking in a wide region and the fusion region of the biological tissue becomes wider.

If a holder provided at a distal portion of aforesaid catheter and including a plurality of lumens in which aforesaid needle member is inserted into is formed such that the shapes of aforesaid plurality of lumens are to be expanded mutually toward the distal direction and aforesaid needle members are moved forward and backward along aforesaid lumen shapes, it becomes possible to expand/contract the distal side of the stick portion by protruding and pulling back the needle member along the lumen shape.

If aforesaid plurality of needle members is constituted with shapes expanding mutually toward the distal direction, and aforesaid needle members in a state in which expansion is closed elastically are made to be housable with respect to a holder provided at a distal portion of aforesaid catheter and including a lumen into which aforesaid stick portion is inserted and into aforesaid lumen, it becomes possible to expand/contract the distal side of the stick portion depending on the elastic force by protruding and pulling back the needle member along the lumen.

If the distal edge of aforesaid stick portion is branched into a plurality of portions and the branch portions expanded mutually toward the distal direction constitute the needle members, and aforesaid needle members in a state in which a branch is closed are made to be housable with respect to a holder provided at a distal portion of aforesaid catheter and including a lumen into which aforesaid stick portion is inserted and into aforesaid lumen, it becomes possible to expand/contract the distal side of the stick portion depending on the elastic force by protruding and pulling back the needle member along the lumen.

If aforesaid stick portion is made by a shape memory material, and aforesaid plurality of needle members expand each other by a shape-recovery temperature higher than the use environment temperature, it becomes possible to expand/contract the distal side of the stick portion by changing the temperature of the stick portion.

If aforesaid stick portion is made by a shape memory material and the distal tip thereof is branched into a plurality of portions and the branch portions are expanded mutually toward the distal direction constituting aforesaid needle members, aforesaid needle members are held in a state in which the branch is closed at the use environment temperature, and the needle member comrades expand by a shape-recovery temperature higher than the use environment temperature, it becomes possible to expand/contract the distal side of the stick portion provided in a manner of being branched into the plurality of portions by changing the temperature of the stick portion.

If aforesaid stick portion made by a shape memory material is heated to the shape-recovery temperature by supplying electric energy from aforesaid electric energy supply means to aforesaid stick portion, it is possible to change the temperature of aforesaid stick portion easily by using, for example, the electric energy supply means provided for mutually fusing the foramen ovale valve and the atrial septum secundum which are biological tissues.

If aforesaid needle member is deformed before the clamping in a direction apart with respect to the sandwich member, is for example curved in an arc shape or is formed in a shape such as a symbol "<", it becomes easy to protrude the needle member in the side direction more only by moving the needle member in the axial direction of the catheter.

If a distal portion of a guiding catheter for guiding aforesaid catheter is bent, regardless of a state of the foramen ovale valve and the atrial septum secundum which differ depending on a person, it is easier for aforesaid catheter to be directed to the foramen oval (opening portion formed at biological tissue) between the foramen ovale valve and the atrial septum secundum easier than a case of a straight shape, and safety, convenience and speediness of the procedure are improved.

If there is provided at the proximal portion of aforesaid catheter with an operation unit at hand for operating aforesaid clamping means, and aforesaid operation unit at hand and aforesaid clamping means are coupled by an operation member inserted into aforesaid catheter, it is possible to operate the clamping means only by the operation unit at hand without moving aforesaid whole catheter forward and backward, it is possible to execute the operation of the sticking and the sandwiching more easily and also accurately and it is possible to execute also the sticking or sandwiching operation by a so-called single hand operation, and the operability is improved more.

### BEST MODE FOR CARRYING OUT THE INVENTION

Hereinafter, exemplified embodiments of the present invention will be explained in detail with reference to the drawings.

### <First Exemplified Embodiment>

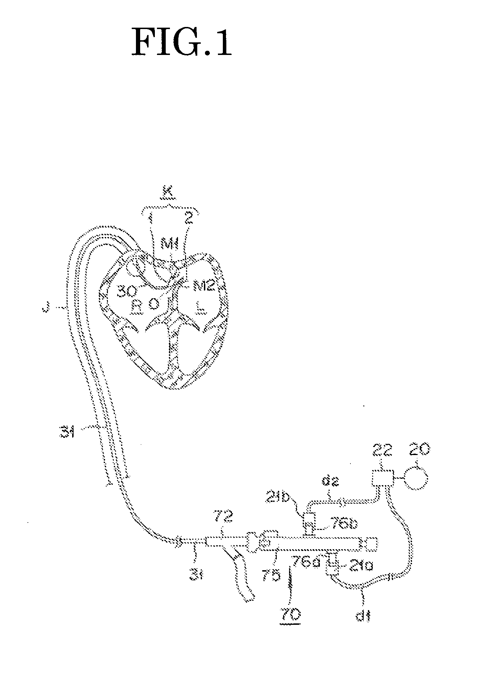

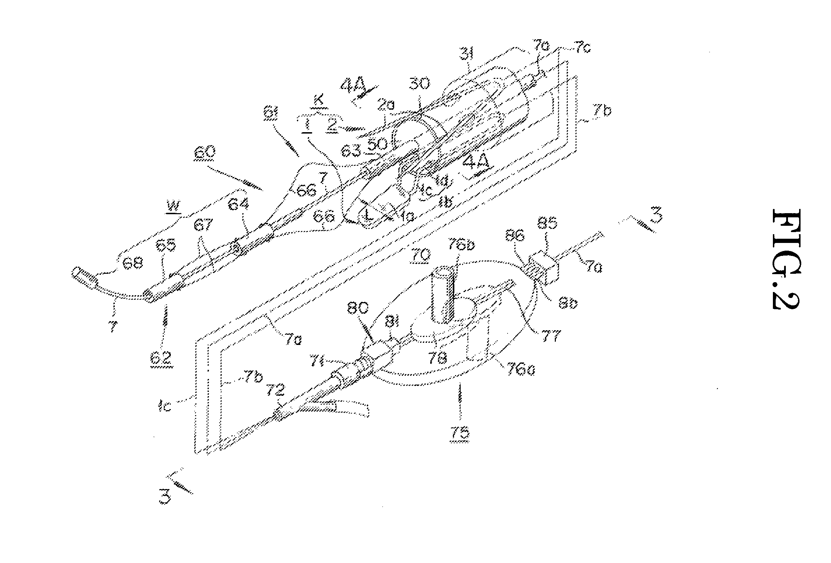

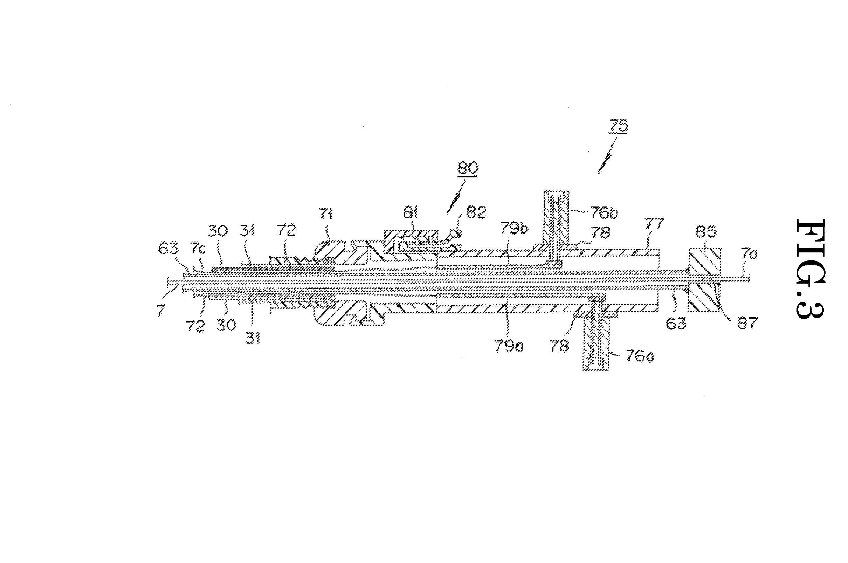

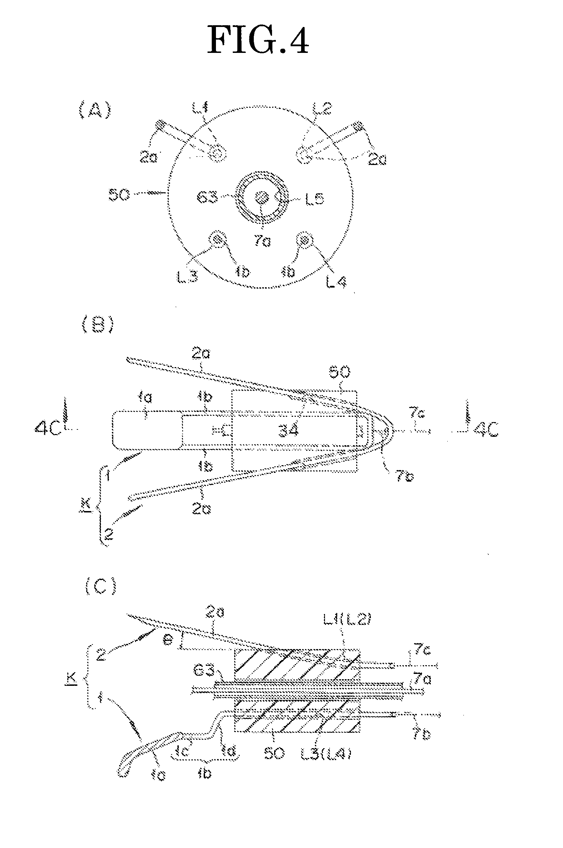

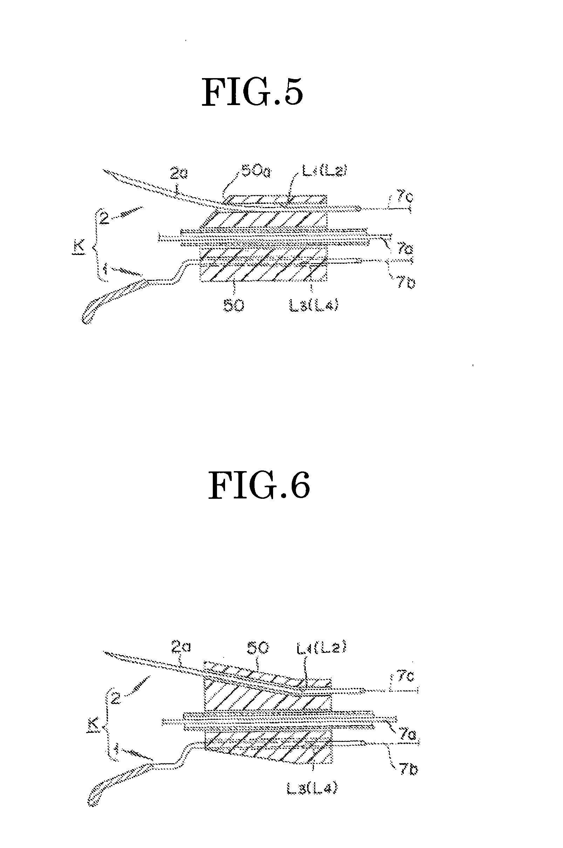

FIG. 1 is a schematic cross-section diagram showing a PFO closing device according to a first exemplified embodiment of the present invention, FIG. 2 is a perspective view of a main portion showing one example of the same device, FIG. 3 is a cross-section diagram along a 3-3 line in FIG. 2, FIG. 4A is a diagram equivalent to the cross-section along a 4A-4A line in FIG. 2, FIG. 4B is a diagram equivalent to the plan view of FIG. 4A, FIG. 4C is a diagram equivalent to the cross-section along a 4C-4C line in FIG. 4B, FIG. 5 is a cross-section diagram showing a modified example of a holder and FIG. 6 is a cross-section diagram showing another modified example of a holder. It should be noted in FIG. 2 that only an operation unit at hand 70 is described by a state of being demagnified because of space limitations.

First, it is outlined with respect to a PFO closing device of the present exemplified embodiment. This device, as shown in FIG. 1, includes clamping means K for sandwiching a foramen ovale valve M2 and an atrial septum secundum M1; and an energy supply means 20 for supplying energy by which a biological tissue M (general term of M1, M2) of a portion sandwiched by the clamping means K is welded or fused, wherein the clamping means K is installed in a percutaneous catheter 30 from a distal tip thereof so as to be protrudable and backward-movable.

This device, when being used, first, is inserted, for example, from a femoral vein J in a state in which there is housed in a guiding catheter 31 the whole the clamping means K which is provided at the distal tip of the catheter 30. If the distal tip reaches the region of the heart at which the procedure is executed, the clamping means K is protruded from the distal tip of the guiding catheter 31 and there are sandwiched by the clamping means K the tissues of the atrial septum secundum M1 and the foramen ovale valve M2 of the heart having the defect O of the foramen ovale. In this sandwich state, the clamping means K is supplied with electric energy, both the tissues are welded and fused and the defect O is closed. It should be noted in the drawing that ^{┌}L_{┘} denotes a left atrium and ^{┌}R_{┘} denotes a right atrium.

This device, as shown in FIGS 1 and 2, includes an operation unit at hand 70 provided on the proximal side; a guiding catheter 31 mounted on the operation unit at hand 70 for the proximal tip thereof; a catheter 30 provided in the guiding catheter 31, clamping means K provided at a distal portion of the catheter 30; and positioning hold means 60 for executing the procedure stably and accurately by the clamping means K. It should be noted in the following explanation that the side of the operation unit at hand of the device is referred to as ^{┌}proximal side _{┘} and the side of the clamping means K or the foramen ovale valve M2 is referred to as ^{┌}distal side_{┘}.

It will be described in more detail. The clamping means K of the present exemplified embodiment is constituted, as shown in FIG. 2, by a sandwich member 1 directly contacting with one side surface of the atrial septum secundum M1 and a stick portion 2 which is stuck into the foramen ovale valve M2. The sandwich member 1 and the stick portion 2 function as electrode members respectively and the proximal portions thereof are held by a holder 50 provided at the distal portion of the catheter 30 and are constituted protrudable from the holder 50.

The sandwich member 1 is constituted by a main body portion 1a including generally a flat plate shape and a predetermined width L; and a pair of wire portions 1b connected to the proximal portion, one piece of operation member 7b is connected on the proximal side of the wire portion 1b and by making the operation member 7b move forward and backward in the axial direction, the sandwich member is displaced so as to protrude from the distal tip of the catheter 30 or so as to approach toward the stick portion 2 side.

The wire portion 1b includes a bend portion 1c and a straight-shaped portion 1d, the straight-shaped portion 1d is inserted into lumens L3, L4 (see FIG. 4) of the holder 50 so as to be movable forward and backward, so that if the operation member 7a is traction-operated, it is possible, when the bend portion 1c gets into the entrance portion of the lumens L3, L4 of the holder 50, to displace the sandwich member 1 so as to approach and separate with respect to the stick portion 2 and it is possible to carry out the sandwich of the biological tissue by both the electrode members easily and smoothly even in case of the distal portion of the fine catheter 30. Also, it is allowed for the wire portion 1b and the operation member 7b to be constituted by one piece of wire.

With respect to the sandwich member 1, a SUS material may be used for a material of the main body portion 1a thereof, in which it is preferable to use a material which does not exert bad influence to a living body such as, for example, gold, silver, platinum, tungsten, palladium or alloys of these, Ni-Ti alloy, titanium alloy and the like.

The stick portion 2 is held by lumens L1, L2 formed in the holder 50 so as to be movable forward and backward and the distal portion thereof is constituted so as to be retractable from the holder 50 by operating an operation member 7c connected to the proximal side. More specifically, each of the stick portion 2 and the sandwich member 1 of the clamping means K is constituted to be movable independently in the axial line direction with respect to the catheter 30, but the stick portion 2, differently from the sandwich member 1, is retractable from the holder 50 and can be made to protrude freely at a desired position. Also, it is allowed for the wire portion 1b and the operation member 7b to be constituted by one piece of wire.

By making each of the stick portion 2 and the sandwich member 1 of the clamping means K movable independently with respect to the catheter 30 by using the operation members 7b, 7c in this manner, it becomes possible to stick the stick portion 2 at an arbitrary position, so that it is possible to execute the procedure very smoothly, safely and also certainly.

In particular, the stick portion 2 of the present exemplified embodiment is constituted by two pieces of needle member 2a, 2a and as shown in FIG. 4B, it is formed such that both the needle members 2a, 2a are expanded in a way of widening mutually. If it is a shape expanded in a way of widening, it happens that the foramen ovale valve M2 is to be stuck into in a wider region and the sandwich region with respect to the sandwich member 1 is to be expanded.

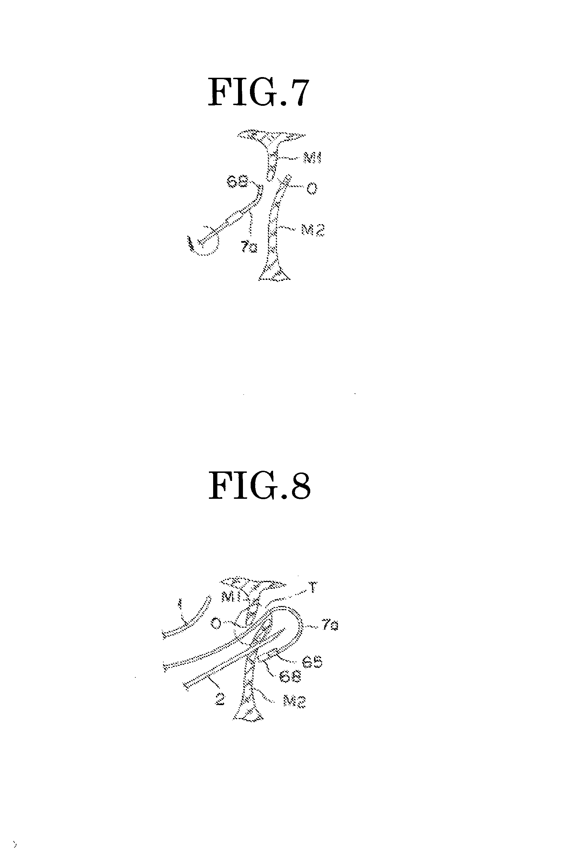

Also, it is also allowed for both the needle members 2a, 2a to be constituted so as to move in/out from the distal region of the catheter 30 in a tilting manner with respect to the axial line of the catheter 30, in other words, such that both the needle members 2a, 2a will move in/out from the side direction of the catheter 30. Therefore, for example, to explain it with reference to FIG. 8, it is ideal for the sticking position to be a foramen ovale valve portion in close vicinity of the position at which the foramen ovale valve M2 and the atrial septum secundum M1 are overlapped (T portion shown by alternate long dashed short dashed line), so that if it is approached to such a sticking position, it becomes possible to execute the sticking by aiming at this.

The present device includes positioning hold means 60 (described in detail later) provided with a main tube 63 for being inserted into a large lumen L5 at the center of the holder 50 and a main operation rod 7a, and before using the sandwich means K, the stick portion 2 is positioning-held by using the positioning hold means 60 and in this case, the sticking is executed in a state in which the main operation rod 7a is inserted into the foramen ovale O beforehand. In other words, the stick portion 2 is configured so as to stick into the foramen ovale valve portion by passing through a passway which is restricted a certain degree by the main operation rod 7a.

If the stick portion 2 is operated in such a state, there is a fear that the sticking can be carried out only for the front side of catheter 30, but if the needle members 2a, 2a can be protruded to the side direction similarly as the present exemplified embodiment, it is naturally directed toward a closed position of the ideal overlapping position T as the distal tip of the catheter 30 approaches the foramen ovale O and moreover, it is possible for the stick portion 2 to execute the sticking action independently, so that the most preferable sticking position is to be chosen and the sticking becomes possible at that position.

For means for retraction from the side direction, as shown in FIG. 4A to FIG. 4C, lumens L1, L2 for the stick portion which are formed in the holder 50 are formed so as to tilt toward the side surface of the distal region from the proximal side end surface and both the needle members 2a, 2a are guided by the lumens L1, L2 so as to protrude in the side direction of the catheter 30. It is allowed for the tilt angle θ of this tilt portion 34 to be set to any value, but generally, around 10° to 30°is preferable and according to circumstances, it is also possible to set it around 90°.

Also, according to circumstances, although it is not shown in the drawing, it is also allowed for both the needle members 2a themselves to be curved in arc shapes in the direction apart with respect to the sandwich member 1 or to be bent in symbol " < " shapes. If these are the curved or bent needle shapes, it becomes easy to protrude both the needle members 2a to the side direction more by only moving both the needle members 2a to the axial direction of the catheter 30.

It is also allowed to change not only shapes of both the needle members 2a themselves but also a shape of the holder 50. For example, as shown in FIG. 5, a cut-out portion 50a is formed at the distal tip of the holder 50 and it is also allowed for a portion of the lumens L1, L2 to move both the needle members 2a easily in the side direction, as shown in FIG. 6, the holder 50 itself is deformed and it is also allowed for both the needle members 2a to be tilted so as to move easily in the side direction.

The stick portion 2 of the present exemplified embodiment has, as shown in FIG. 2, a structure in which very fine two pieces of needle members 2a, 2a whose sections perpendicular to the axes are circles and whose front ends are sharply pointed are arranged by being mutually expanded and if the foramen ovale valve M2 is stuck into by such a stick portion 2, as the region covered by the two pieces of needle members 2a, 2a is wide, it is possible, even for various forms of the foramen ovale valve and the atrial septum secundum, to execute positioning of at least one electrode member to the foramen ovale valve M2 and the sandwich operation of the biological tissue M becomes easy. It is also allowed for the sections perpendicular to the axes to be in polygonal shapes.

Solid cylinders are not always necessary for the needle members 2a, 2a and it is allowed to employ hollow cylindrical shaped members and it is preferable for the outer diameter thereof to be around 0.1mm to 2mm in order to be installed in the catheter 30. For a material, a SUS material is used, but it is also possible to use a material which does not exert bad influence to a living body such as, for example, gold, silver, platinum, tungsten, palladium, titanium or alloys of these and the like. Although it is not limited in particular, it is enough if the mutual distance of two pieces of the electrode portions 3a, 3a is selected such that the foramen ovale valve M2 and the atrial septum secundum M1 can be sandwiched in a certain range, and also with respect to the number of pieces, it may be not only two pieces but also a larger number of pieces than two.

As the operation members 7b, 7c for moving the sandwich member 1 and the stick portion 2 in/out from the catheter 30, any kinds of members can be employed if they are fine wire-shaped members, can move the clamping means K forward and backward in the catheter 30 and also are members through which electric current can flow, and it is preferable to use a wire such as, for example, stainless, Ni-Ti, titanium and the like. Both the operation members 7b, 7c are inserted into the catheter 30 and are connected with the energy supply means 20 through operation levers 76a, 76b, connection members 21a, 21b, conductive wires d1, d2 and a control unit 22 which will be described later.

It should be noted that the holder 50 includes, as shown in FIG. 4A, a plurality of lumens L1 to L5, but it is also allowed to constitute these respective lumens to L5 by catheters respectively.

As shown in FIG. 1 to FIG. 3, the operation unit at hand 70 includes a handle member 75 composed of a flat member such that the surgery operator can grasp it by a single hand. On the surface side (upper surface side) of the handle member 75, there is protruded the operation lever 76b coupled with the proximal side of the operation member 7c of the stick portion 2 and on the rear surface side (lower surface side) of the handle member 75, there is protruded the operation lever 76a for operating the operation member 7b of the sandwich member 1.

The operation levers 76a, 76b are provided slidably in a slide groove 77 formed on the handle member 75 and a brim portion 78 is provided at the lower end portion, in which it is configured to be able to slidingly move smoothly without falling down.

The operation members 7b, 7c are wired until the upper end portions of the operation levers 76a, 76b and it is constituted such that connection members 21a, 21b composed of sockets, couplers or the like which are provided at the distal tips of the conductive wires d1, d2 from the electric energy supply means 20 and the upper end portions of the respective operation levers 76a, 76b are to concave-convex-fit detachably and owing to this fit, the electric energy supply means 20 and the operation levers 76a, 76b are in an electrically conductive state.

Consequently, after the procedure concerning the sticking and the sandwich caused by both the operation levers 76a, 76b is completed, if the connection members 21a, 21b are connected to the respective operation levers 76a, 76b, it is possible to supply electric energy by which the foramen ovale valve M2 and the atrial septum secundum M1 are fused. In other words, not only is it possible to supply electric energy at a desired time point but it is also possible to execute the procedure accurately and also smoothly in case of executing the procedure without any fact that the conductive wires d1, d2 will become obstructive.

It is allowed to directly connect the respective operation members 7b, 7c with the respective operation levers 76a, 76b by brazing or the like, but in the present exemplified embodiment, there are provided slide pieces 79a, 79b so as to face the slide groove 77 formed on the handle member 75, the proximal sides of the respective operation members 7b, 7c are brazed on the distal tips of the slide pieces 79a, 79b and the inner ends of the respective operation levers 76a, 76b are brazed on the proximal tips of the slide pieces 79a, 79b. By doing like this, not only the operability of the device is improved but also the strength or the durability is to be improved and it is preferable. It should be noted that the slide pieces 79a, 79b are provided slidably in the groove (not shown) formed on the handle member 75.

In addition, the operation unit at hand 70 of the present exemplified embodiment also includes a lock mechanism 80 for locking a state in which the stick portion 2 is protruded from the distal portion of the catheter 30 at the distal portion of the handle member 75.

The lock mechanism 80 has a constitution in which there is provided a bump member for restricting the sliding movement of the operation lever 76b for the needle on the distal side of the slide groove 77 formed on the handle member 75 and the sliding movement is locked by pressing the operation lever 76b for the needle to the bump member. If the stick portion 2 is locked in a state of being protruded from the distal portion of the catheter 30, it is possible to supply electric energy while maintaining the protrusion state of the stick portion 2, it never happens that the stick portion 2 is deviated at the time of supplying electric energy and it is possible to carry out the procedure more stably and also accurately.

It is also allowed for the lock mechanism 80 to employ any form of mechanism if it is possible to lock the operation lever 76b for the needle, but as shown in FIG. 3, if a tubular block 81 is fixed on the handle member 75 and a slide member 82 is provided movably in the tubular block 81, it is possible to adjust the fixed position of the operation lever 76b for the needle and it is preferable.

The energy supply means 20 is a unit for supplying electric energy to the clamping means K and a well-known system constitution is employed, so that the detailled description is avoided, but if easiness of control is taken into account, it is preferable to employ an electrical power supply regardless of a direct-current power supply or an alternate-current power supply. However, it is also allowed to employ not only this kind but also any kind of supply means if it can supply energy in which the foramen ovale valve M2 and the atrial septum secundum M1 which are sandwiched by the clamping means K are melted by the heat and are pressed and fixed by an adhesive agent such as collagen, erastin and the like. For example, it is also possible to use a super sonic wave, laser, microwave or high frequency and the like.

Also, it is possible for an electric energy supply system to use a monopolar system which energizes between the stick portion 2 or the sandwich member 1 of the right atrium R side and the counterpart pole plate provided at a backbone portion, a bipolar system which energizes between the sandwich member 1 of the right atrium R side and the stick portion 2 of the left atrium L side and the like. In particular, if it is formed as a bipolar system for controlling the current by the impedance of the biological tissue between the stick portion 2 and the sandwich member 1, there are advantages that it can have correspondence easily in response to the state of the tissues of the foramen ovale valve M2 and the atrial septum secundum M1 which differ depending on a person and the safety and the convenience of the procedure are obtained.

Five lumens L1 to L5 are established in the holder 50 as shown in FIG. 4, and the stick portion 2 and the sandwich member 1 are inserted-into the first and second lumens L1, L2 and the third and fourth lumens L3, L4 as mentioned above and the positioning hold means 60 is provided in the fifth lumen L5 having the maximum aperture which exists at the center.

The positioning hold means 60, as shown in FIG. 2, generally, includes a positioning portion 61 for positioning the stick portion 2 with respect to a foramen ovale O and a holding portion 62 for holding the foramen ovale valve M2 with respect to the sticking direction of the stick portion 2 so as not to allow the backward movement thereof, and normally it is housed in the guiding catheter 31, but it is pushed out from the guiding catheter 31 by the main operation rod 7a and the main tube 63 when using it.

To describe it in more detail, there is provided in the center lumen L5 a main tube 63 provided for pulling and withdrawing the positioning hold means 60 in/from the catheter and for purpose of aiming at reinforcement of the catheter 30; and an main operation rod 7a which is provided so as to move forward and backward freely in the axial direction in the main tube 63.

There is provided at the distal portion of the main tube 63 the positioning hold means 60 including a positioning portion 61 which is operated so as to expand or contract by the operation of the main operation rod 7a and constituted by a pair of a first elastic wires 66 coupling the main tube 63 and a middle sleeve body 64; and a hold portion 62 which includes a bump member 68 provided at the distal portion of the main operation rod 7a, a distal tip sleeve body 65 and a pair of a second elastic wires 67 coupling the middle sleeve body 64 and the distal tip sleeve body 65, and which holds the foramen ovale valve M2 by the bump member 68 and the distal tip sleeve body 65.

With respect to the positioning portion 61, the main operation rod 7a is protruded from the distal tip of the main tube 63, the first elastic members 66 are displaced outward by the operation of moving the main operation rod 7a forward and backward in the axial direction, respective first elastic members 66 press an inner fringe of the foramen ovale O with approximately equal elastic forces, and the stick portion 2 is center-aligned with respect to the foramen ovale O. In other words, it exhibits a function for positioning the stick portion 2 which is positioned between both the first elastic members at the center portion of the foramen ovale O.

The holding portion 62 includes a bending mechanism W for bending the distal portion of the main operation rod 7a by operating the main operation rod 7a in the axial direction so as to move forward and backward. The bending mechanism W bends the holding portion 62 so as to face the direction in which the stick portion 2 sticks the foramen ovale valve M2 and exhibits a function for holding the foramen ovale valve M2. Here, the bending mechanism W is constituted by the middle sleeve body 64, the distal tip sleeve body 65, the second elastic wire 67 for coupling both the sleeve bodies 64, 65 and the bump member 68.

The proximal tip of the first elastic wire 66 is welded on the distal tip of the main tube 63 and the distal side thereof is welded on the middle sleeve body 64. On the other hand, the proximal tip of the second elastic wire 67 is welded on the distal tip of the middle sleeve body 64 and the distal side thereof is welded on the distal tip sleeve body 65.

It is preferable for a specific example of the first and second elastic wires 66, 67 to use a metallic wire such as stainless steel, nickel-titanium, super elastic alloy (for example, Ni-Ti alloy) and the like with an outer diameter of around 0.1mm to 0.5mm. It is also allowed to prevent the tissue from being wounded by coating a metallic wire with a (soft) resin tube.

The holding portion 62 has a constitution in which the first elastic wire 66 of the proximal side bends prior to the second elastic wire 67 of the distal side; the positioning of the stick portion 2 is executed; subsequently, the main operation rod 7a itself is deformed accompanied by the bump member 68 and the distal tip sleeve body 65; and the positioning portion 61 holds the foramen ovale valve M2 after positioning the stick portion 2.

For such a constitution, for example, it is also possible to use such methods as a method which uses the second elastic wire 67 having higher stiffness materially than that of the first elastic wire 66; and a method in which an easily-deformable portion is formed by bending a portion of the first elastic wire 66 beforehand or the like and when traction force is applied, the first elastic wire 66 is bent previously compared with the second elastic wire 67 by the deformation of the easily-deformable portion.

When doing in this manner, the first elastic wire 66 of the proximal side is attached to an inner fringe of the foramen ovale O only by the traction of the main operation rod 7a backward and the positioning of the stick portion 2 can be executed, and when applying further traction, the second elastic wire 67 of the distal side is protruded and deformed like an arc shape toward the outward direction in the radial direction and it is possible to hold the foramen ovale valve M2 so as not to allow the backward movement thereof such that it becomes easy for the stick portion 2 to be stuck.

Also, the main operation rod 7a is configured to be 360° rotatable centering around the axial line in the main tube 36. If the main operation rod 7a is 360°rotatable, when the distal tip of the main operation rod 7a is inserted up to the vicinity of the foramen ovale O, it is possible to position-change the main operation rod 7a rotatingly and even if a state of the foramen ovale O is deformed variously, it is possible, regardless of the shape state thereof, to insert the distal tip of the device into the foramen ovale O and it is possible not only to simplify the procedure but also to execute it speedily.

There is provided at the rear end portion of the handle member 75 with a handle member for main tube 85 which moves the main tube 63 forward and backward through two pieces of slide rails 86. The proximal side of the main tube 63 is firmly fixed at the handle member for main tube 85, so that when the handle member for main tube 85 is pulled toward the direction apart from the handle member 75, in other words, when it is pulled backward, it is possible to pull the main tube 63 into the center lumen L5 of the catheter 30 and according to this, it is possible to withdraw the whole positioning hold means 60 into the catheter 30.

It should be noted that in order to configure the main operation rod 7a to be 360°rotatable, the main operation rod 7a is inserted into a through-hole 87 of the handle member for main tube 85 and is extended.

For the material constituting this main tube 63, it is possible to use a deformable elastic material such as, for example, polyimide resin, polyurethane, PET, nylon, fluorine resin, polypropylene and the like.

Also, it is also allowed for the main operation rod 7a to employ any kind of rod if it is a fine hollow cylindrical wire and possesses a comparison suitability, but it is preferable to use a fine tube such as, for example, stainless, Ni-Ti, titanium and the like.

A Y connector 72 into which a contrast medium or the like can be injected is coupled to the distal tip of the handle member 75 through a coupling member 71 and the proximal tip of the catheter 30 and the end portion of the guiding catheter 31 are held at the coupling member 71.

With respect to the guiding catheter 31 of the present exemplified embodiment, the distal tip thereof is bent gently in an arc shape in order to make it easy to be inserted into the foramen ovale O between the foramen ovale valve M2 and the atrial septum secundum M1. The foramen ovale valve M2 and the atrial septum secundum M1 differ depending on the person, so that when the distal tip of the guiding catheter 31 is bent, the guiding catheter 31 itself is rotatingly moved and it is possible to insert the guiding catheter 31 into the foramen ovale O at a position in which the insertion becomes easiest, and safety and convenience of the procedure are improved compared with the case of a straight shape.

Next, the operation of the present exemplified embodiment will be explained.

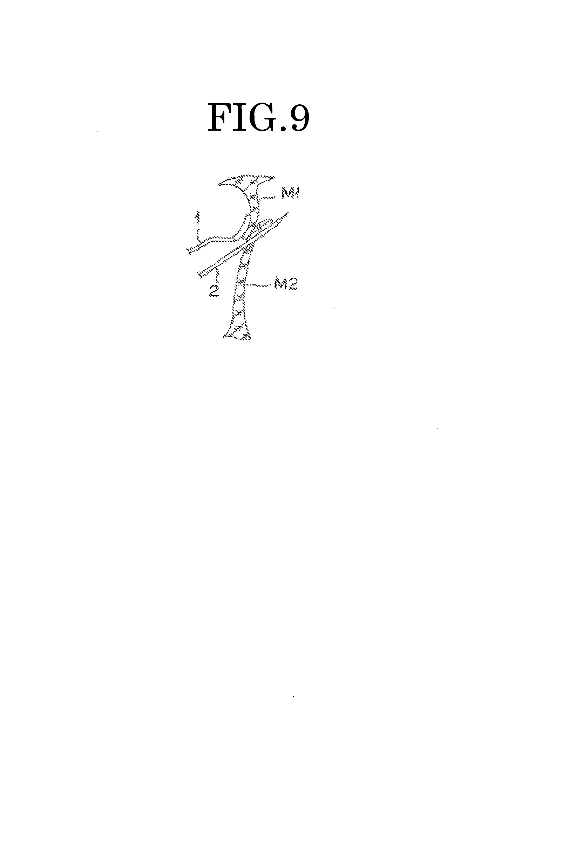

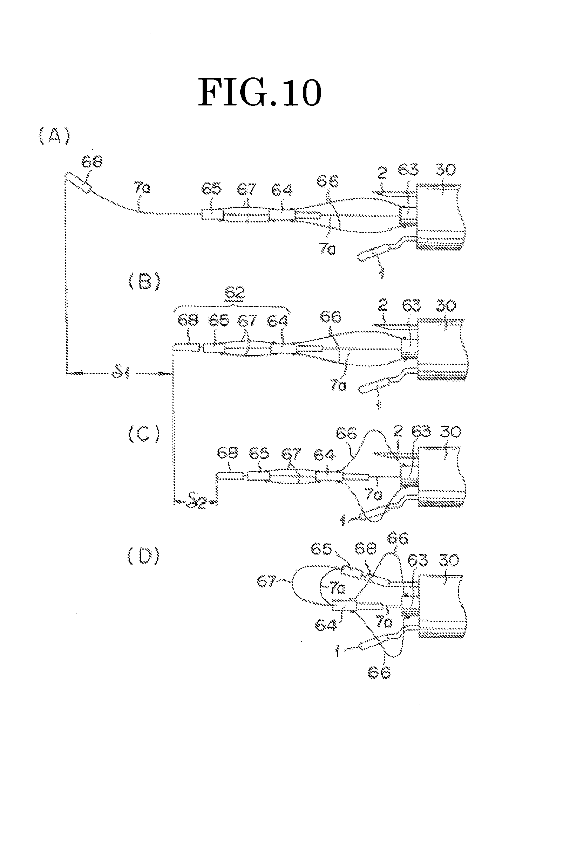

FIG. 7 is a cross-sectional outlined view in which a main operation rod is inserted into the foramen ovale, FIG. 8 is a cross-sectional outlined view of a state in which a foramen ovale valve is held and a needle portion is stuck thereto, FIG. 9 is a cross-sectional outlined view in which a foramen ovale valve and an atrial septum secundum are sandwiched by a needle portion and a sandwich member and FIG. 10A to FIG. 10D are outlined views showing operation states of a distal portion of a PFO closing device. It should be noted in FIG. 10A to FIG. 10D that shapes and positions of the second elastic wire 66 are drawn approximately in flush with the sandwich member 1 and the stick portion 2, but in order to facilitate the understanding, they are shown by states in which the positions are shown by being displaced by 90°and those are different from the actual deformation states.

First, the surgery operator moves the handle member for main tube 85 of the operation unit at hand 70 backward with respect to the handle member 75 and makes a state in which the sandwich member 1, the electrode portions 3a, 3a and the like are housed in the guiding catheter 31, and in this state, based on the common procedure, the distal tip of the guiding catheter 31 is inserted from a predetermined position of the living body by making a guide wire as a guide thereof and it is reached until the right atrium R by passing through the femoral vena cave J. Here, it is also allowed to insert only the guiding catheter 31 into the living body and afterward to insert the catheter 30 by making that guiding catheter 31 as a guide.

When the distal tip of the guiding catheter 31 reaches the right atrium R, as shown in FIG. 7, the catheter 30 is pushed out and moved toward the foramen ovale O between the foramen ovale valve M2 and the atrial septum secundum M1. The distal tip of the guiding catheter 31 is curved, so that it is possible to move it toward the foramen ovale O comparatively easily.

Next, the main operation rod 7a is moved forward and, as shown in FIG. 10A, the distal tip of the main operation rod 7a is protruded from the distal tip sleeve body 65 and inserted into the left atrium L. It is possible to observe this protruding state from the outside by eyes if a marker is provided on the bump member 68 or the like, and it is possible to sensuously identify the position of the distal tip of the main operation rod 7a when the distal tip of the main operation rod 7a is bumped with an inner wall of the left atrium L or the like according to this protrusion even in a case in which it is difficult to observe by eyes. In the present exemplified embodiment, the main operation rod 7a is made to be rotatable by 360°, so that, as shown in FIG. 7, when the main operation rod 7a is moved forward while rotating, it is possible to insert it into the foramen ovale O easily.

After the identification of the distal position of the main operation rod 7a, as shown in FIG. 10B, the main operation rod 7a is moved backward until the bump member 68 of the main operation rod 7a attaches to the distal tip sleeve body 65 (amount of backward movement is ┌51┘ in FIG. 10B). Then, the handle member 75 is operated, and the second elastic wire 67, the sandwich member 1 and the stick portion 2 are positioned in the vicinity of the foramen ovale valve M2 and the whole holding portion 62 is inserted into the left atrium L side.

When the main operation rod 7a is further moved backward (amount of backward movement is ^{┌}62_{┘} in FIG. 10C), the operation force for the backward movement is transmitted by the main operation rod 7a to the first elastic wire 66 firmly fixed on the distal tip of the main tube 63 through the bump member 68, the distal tip sleeve body 65, the second elastic wire 67 and the middle sleeve body 64, and the first elastic wire 66 is, as shown in FIG. 10C, protruded and deformed in an arc shape toward the outside direction in the radial direction. However, at this time point, the second elastic wire 67 is not deformed.

Consequently, it happens that the first elastic wire 66 is deformed while pushing and widening an opening edge portion of the foramen ovale O, so that the stick portion 2 which is provided in close vicinity of the first elastic wire 66 is center-aligned with respect to the foramen ovale O and the stick portion 2 is positioned at the center of the foramen ovale O.

Further, as shown in FIG. 10D, the main operation rod 7a is operated so as to move backward and when a rear end of the middle sleeve body 64 is attached to the distal tip of the main tube 63, the first elastic wire 66 is not deformed so much and the second elastic wire 67 of the distal side is protruded and deformed in an arc shape toward the outside direction in the radial direction by the above-mentioned operation force. Consequently, as shown in FIG. 8, in the left atrium L, the bump member 68 and the distal tip sleeve body 65 approach to the stick portion 2, so that the bump member 68 and the distal tip sleeve body 65 are attached to the surface of the left atrium side of the foramen ovale valve M2 and hold this.

In this state, the operation lever 76b for the needle is moved forward until it is attached to the lock mechanism 80, the stick portion 2 is protruded from the side portion of the catheter 30 through the operation member 7c and when the operation lever 76b for the needle is moved forward, it is possible to execute the sticking at an ideal position in the vicinity of the overlapping position of the atrial septum secundum M1 and the foramen ovale valve M2. On an occasion of this sticking, as shown in FIG. 8, it becomes in a state in which the atrial septum secundum M1 and the foramen ovale valve M2 exist between the sandwich member 1 and the stick portion 2.

There is no fear that the position of the stick portion 2 is deviated, because the operation lever 76b for the needle is attached to the lock mechanism 80 and also, when the stick portion 2 is stuck once, the position of the stick portion 2 is position-fixed with respect to the relation with the foramen ovale valve M2, that is, it happens that the positioning is to be carried out. Therefore, it is possible for the surgery operator to concentrate on the sandwich by the sandwich member 1 without considering about a state of the stick portion 2, and the sandwich operation also becomes easy.

When the operation lever 76a for the sandwich member is moved forward, the sandwich member 1 is protruded from the distal tip of the catheter 30 through the operation member 7b, the operation member 7b presses the atrial septum secundum M1 toward the foramen ovale valve M2, and the atrial septum secundum M1 and the foramen ovale valve M2 are fixed for the positions thereof in the wall thickness direction, that is, in the forward/backward direction if being described about the operation state thereof and, as shown in FIG. 9, it becomes in a state in which the atrial septum secundum M1 and the foramen ovale valve M2 exist between the sandwich member 1 and the stick portion 2.

In this step, the main operation rod 7a is returned once and, as shown in FIG. 10B, the first elastic wire 66 and the second elastic wire 67 are made to be in a straight-line shape and thereafter, the handle member for the main tube 85 is operated so as to move backward, the main tube 63 is moved backward, and the whole positioning hold means 60 is withdrawn in the lumen L5 of the catheter 30.

After this withdrawal, when the operation lever 76a for the sandwich member is operated so as to move backward and the sandwich member 1 is moved backward through the operation member 7b, the bend portion 1c of the sandwich member 1 is deformed by the end portion of the holder 50 so as to approach to the stick portion 2 side and, as shown in FIG. 9, the atrial septum secundum M1 and the foramen ovale valve M2 are firmly sandwiched between the sandwich member 1 and the stick portion 2. It should be noted that the sandwich member 1, the positioning hold means 60 and the stick portion 2 can respectively move independently, so that it may not always operated by such a sequence mentioned above. For example, it is also possible to stick the foramen ovale valve M2 by the stick portion 2 after a state in which the sandwich member 1 is moved and the atrial septum secundum M1 is held by the sandwich member 1 and thereafter, the positioning hold means 60 is operated and the atrial septum secundum M1 and the foramen ovale valve M2 are sandwiched by the sandwich member 1 and the positioning hold means 60.

If the operation lever 76b for the needle is pressed to the lock mechanism 80 and a lock state is maintained, a sandwich state of the atrial septum secundum M1 and the foramen ovale valve M2 caused by the sandwich member 1 and the stick portion 2 is maintained. By this lock, there is no fear that the stick portion 2 is deviated, so that if the connection members 21a, 21b are concave-convex-fitted to the operation lever 76b for the needle and the operation lever 76a for the sandwich member, a predetermined electric current controlled by the control unit 22 flows from the operation lever 76b for the needle and the operation lever 76a for the sandwich member into the sandwich member 1 and the stick portion 2 through the operation members 7b, 7c. The atrial septum secundum M1 and the foramen ovale valve M2 are heated by the supply of this electric energy.

By controlling electric energy in the control unit 22, even if a portion of the sandwich member 1 and the stick member 2 is exposed in the blood, it is possible in this step to prevent the thrombus from being attached to the sandwich member 1 and the stick member 2. However, if coating for preventing thrombus attachment is applied on the surfaces of the sandwich member 1 and the stick member 2, it happens that the attachment of the thrombus is prevented more certainly and it is preferable.

When the heating is continued while maintaining a fusion temperature, the tissues of the atrial septum secundum M1 and the foramen ovale valve M2 melt and are fused mutually by an adhesive agent such as collagen, erastin and the like. With respect to the stick portion 2 of the present exemplified embodiment, the expanded plurality of needle members 2a, 2a are contacted with the foramen ovale valve M2 in a wide region, so that it is possible to sandwich the atrial septum secundum M1 and the foramen ovale valve M2 after sticking in a wide region and the fusion region of the biological tissue becomes wider.

When the fusion is completed, the energization is stopped, the operation lever 76b for the needle and the operation lever 76a for the sandwich member are moved backward and are housed in the guiding catheter 31. Then, if the guiding catheter 31 is pulled-out from the living body, the procedure is completed. It should be noted that after the procedure is completed, a very small hole remains on the foramen ovale valve M2 by the pulling-out of the stick portion 2, but it is healed afterward and bad influence such as generation of a thrombus and the like will never occur.

### <Second Exemplified Embodiment>

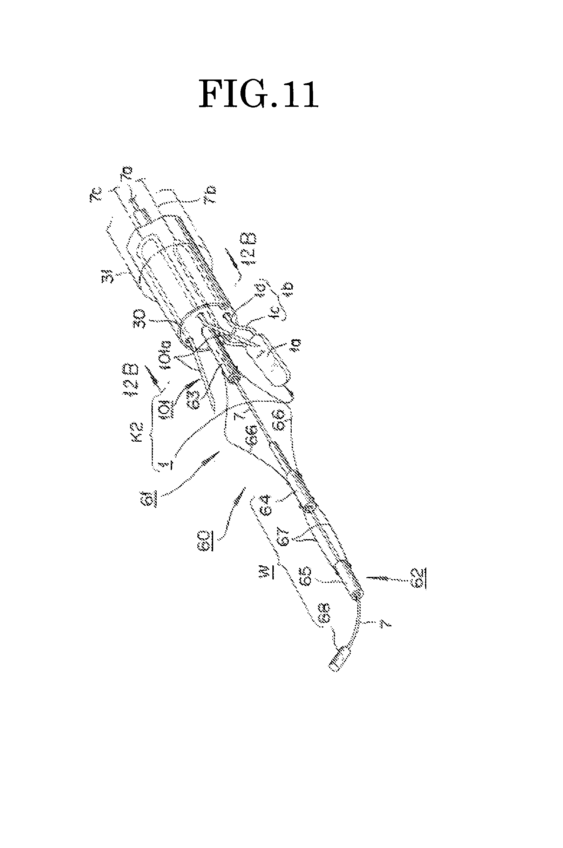

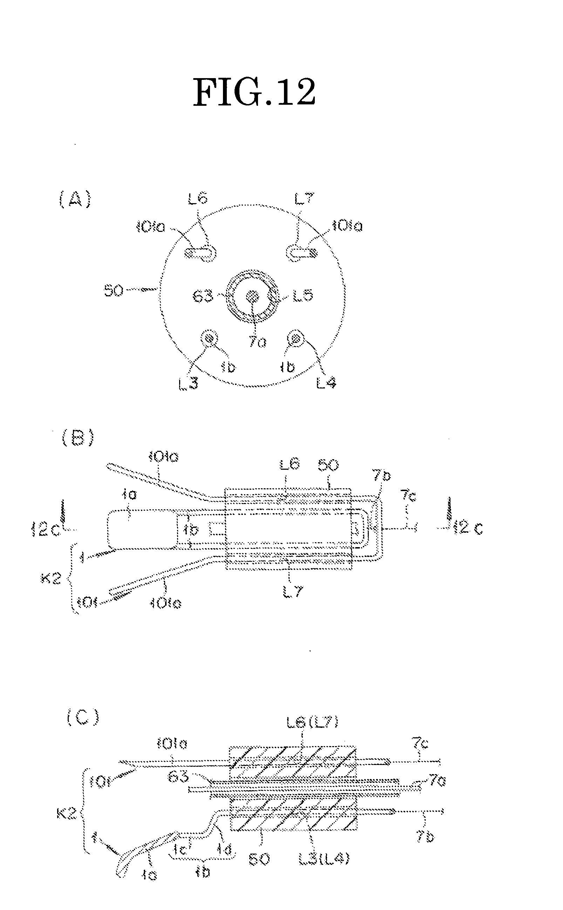

FIG. 11 is a perspective view of a main portion showing a PFO closing device according to a second exemplified embodiment of the present invention, FIG. 12A is a diagram equivalent to a cross-section along a 12A-12A line in FIG. 11, FIG. 12B is a diagram equivalent to a plan view of FIG. 12A and FIG. 12C is a diagram equivalent to a cross-section along a 12C-12C line in FIG. 12B. It should be noted that with respect to the regions having the functions similar to those of the first exemplified embodiment, the same reference numerals are used and the explanation thereof is omitted in order to avoid repetition.

With respect to a PFO closing device according to a second exemplified embodiment, constitutions of a stick portion 101 and lumens L6, L7 for the stick portion are different from those of the PFO closing device according to the first exemplified embodiment.

As shown in FIGS. 11 and 12, clamping means K2 of the second exemplified embodiment is constituted by a sandwich member 1 for contacting directly with one side of the atrial septum secundum M1 and a stick portion 101 to be stuck into the foramen ovale valve M2. The stick portion 101 is constituted by two pieces of needle members 101a, 101a and both the needle members 101a, 101a are formed in shapes expanded in a way of widening mutually toward the distal direction. It should be noted, in the present exemplified embodiment, that both the needle members 101a, 101a are formed by bending in symbol " < " shapes, but it is also allowed for them to be formed by being curved in arc shapes.

There are formed the lumens L6, L7 for the stick portion along the axial direction of the catheter 30. Therefore, in a state in which both the needle members 101a, 101a are housed in the lumens, the bends of both the needle members 101a, 101a are stretched by the elastic deformation and it is in a state in which the expansion is closed. From this state, if the operation lever 76b for the needle is operated and the needle members 101a, 101a are protruded, the bends of both the needle members 101a, 101a are released by the elastic force and it returns to a state in which they are expanded toward the distal side.

Also, if the operation lever 76b for the needle is operated again and both the needle members 101a, 101a are pulled back into the lumen, the bends of both the needle members 101a, 101a are stretched by the elastic deformation and it becomes in a state in which the expansion is closed and they are housed in the lumens.

Depending also on the PFO closing device according to the second exemplified embodiment, there are exhibited operations and effects similar to those of the PFO closing device according to the first exemplified embodiment and the expanded needle members 101a, 101a will contact with the foramen ovale valve M2 in a wide region, so that it is possible to sandwich the atrial septum secundum M1 and the foramen ovale valve M2 after the sticking in a wide region and it is possible to make the fusion region of the biological tissue larger. Also, it is possible to expand/contract the needle members 101a, 101a only by moving the operation lever 76b for the needle forward and backward, so that the operability thereof is excellent.

Here, it is also allowed for the lumens L6, L7 of the second exemplified embodiment to be formed in shapes expanded in a way of widening toward the distal side similarly as the lumens L1, L2 of the first exemplified embodiment. In addition, it is also allowed for the needle members 101a, 101a which constitute the stick portion 101 to be provided by three or more pieces.

### <Third Exemplified Embodiment>

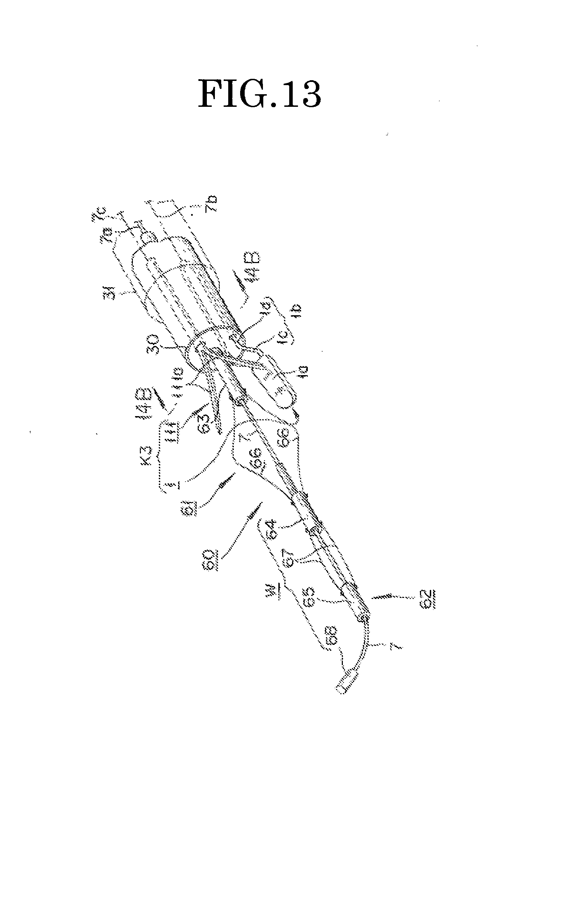

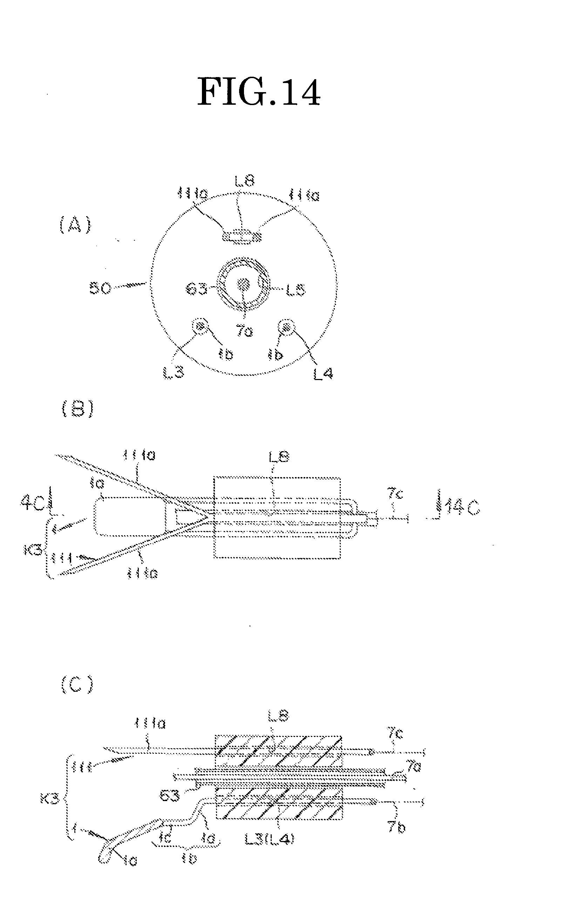

FIG. 13 is a perspective view of a main portion showing a PFO closing device according to a third exemplified embodiment of the present invention, FIG. 14A is a diagram equivalent to a cross-section along a 14A-14A line in FIG. 13, FIG. 14B is a diagram equivalent to a plan view of the FIG. 14A and FIG. 14C is a diagram equivalent to a cross-section along a 14C-14C line in FIG. 14B. It should be noted that with respect to the regions having the functions similar to those of the first and the second exemplified embodiments, the same reference numerals are used and the explanation thereof is omitted in order to avoid repetition.

With respect to a PFO closing device according to a third exemplified embodiment, constitutions of a stick portion 111 and a lumen L8 for the stick portion are different from those of the PFO closing device according to the first exemplified embodiment.

As shown in FIGS. 13 and 14, clamping means K3 of the third exemplified embodiment is constituted by a sandwich member 1 for contacting directly with one side of the atrial septum secundum M1 and a stick portion 111 to be stuck into the foramen ovale valve M2. With respect to the stick portion 111, the distal side thereof is branched into two pieces and the respective branch portions expanded mutually toward the distal direction constitute needle members 111a, 111a.

The lumen L8 for the stick portion is formed along the axial direction of the catheter 30, but it is also allowed to form it in a tilting manner with respect to the axis direction. In a state in which the stick portion 111 is housed in the lumen L8, it becomes in a state in which the expansion of both the needle members 111a, 111a is closed by the elastic deformation and if the operation lever 76b for the needle is operated from this state and the needle members 111a, 111a are protruded, it happens that the bends of both the needle members 111a, 111a are released by the elastic force and return to a state of expansion toward the distal side.

Also, if the operation lever 76b for the needle is operated again and both needle members 111a, 111a are pulled back into the lumen L8, the bends of both the needle members 111a, 111a are stretched by the elastic deformation and it is reached a state in which the expansion of both the needle members 111a, 111a is closed and they are housed in the lumen L8.

Depending also on the PFO closing device according to the third exemplified embodiment, there are exhibited operations and effects similar to those of the PFO closing device according to the first exemplified embodiment and the expanded needle members 111a, 111a will contact with the foramen ovale valve M2 in a wide region, so that it is possible to sandwich the atrial septum secundum M1 and the foramen ovale valve M2 after the sticking in a wide region and it is possible to make the fusion region of the biological tissue larger. Also, it is possible to expand/contract the needle members 111a, 111a only by moving the operation lever 76b for the needle forward and backward, so that the operability thereof is excellent.

It should be noted that the needle members 111a of the of the stick portion 111 of the third exemplified embodiment may be provided by two or more pieces. In addition, the stick portion 111 including the needle member 111a which is branched into a plurality of pieces may be provided by two or more pieces.

### <Fourth Exemplified Embodiment>

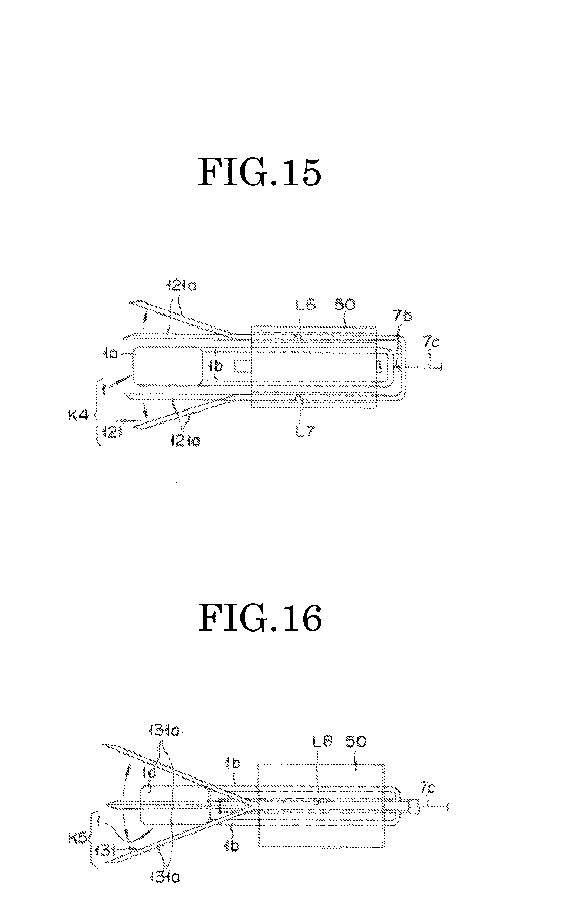

FIG. 15 is a plan view showing a stick portion of a PFO closing device according to a fourth exemplified embodiment of the present invention. It should be noted that with respect to the regions having the functions similar to those of the first to the third exemplified embodiments, the same reference numerals are used and the explanation thereof is omitted in order to avoid repetition.

The constitution of the PFO closing device according to the fourth exemplified embodiment is similar to the PFO closing device according to the second exemplified embodiment except a stick portion 121.

Clamping means K4 of the fourth exemplified embodiment is constituted, as shown in FIG. 15, by a sandwich member 1 for contacting directly to one side of the atrial septum secundum M1 and a stick portion 121 for being stuck into the foramen ovale valve M2. The stick portion 121 of the fourth exemplified embodiment is constituted by two pieces of needle members 121a, 121a and both needle members 121a, 121a are formed by a shape memory metal. There is used, for example, an Ni-Ti alloy for the shape memory metal. In the use environment temperature (temperature in the body), both needle members 121a, 121a are in straight shapes as shown by an alternate long and short dash line in FIG. 15 and are housed in the lumens L6, L7 in this state. The operation lever 76b for the needle is operated from this state and the needle members 121a, 121a are protruded and thereafter, by heating the needle members 121a, 121a to a shape-recovery temperature, they are deformed into the stored shapes which are expanded to the distal side.

The heating of the needle members 121a, 121a is executed by supplying electric energy between the stick portion 121 and the sandwich member 1 from the electric energy supply means 20. It should be noted that the amount of supply of the electric energy at that time is less than the energy necessary for fusing the biological tissue and, for example, the shape-recovery temperature is 40°and the temperature for fusing the biological tissue is 70°.

When the PFO closing device according to the present exemplified embodiment is used, the foramen ovale valve M2 is held by the positioning hold means 60 from the left atrium side and thereafter, the needle members 121a, 121a are protruded from the catheter 30. Next, owing to the electric energy supply means 20, there is supplied electric energy between the stick portion 121 and the sandwich member 1, the stick portion 121 is heated until the shape-recovery temperature and the needle members 121a, 121a of the stick portion 121 are expanded. Next, the lever 76b for the needle protrusion is moved forward and the needle members 121a, 121a are stuck at the aimed position of the foramen ovale valve M2. Thereafter, the atrial septum secundum M1 and the foramen ovale valve M2 are held between the sandwich member 1 and the stick portion 121, electric energy is supplied between the stick portion 121 and the sandwich member by the electric energy supply means 20 and the atrial septum secundum M1 and the foramen ovale valve M2 are thermally fused.

After the thermal fusion is ended, the expansion of the needle members 121a, 121a is closed by stopping the supply of the electric energy and lowering the temperature of the needle members 121a, 121a and the needle members 121a, 121a whose shapes returned to the straight shapes are stored in the catheter 30. Alternatively, it is also possible to close the expansion of the needle members 121a, 121a elastically (or plastically) and to store them by pulling the needle members 121a, 121 of the expanded state into the lumens L6, L7 of the catheter 30.

It should be noted in the present exemplified embodiment that the expansion of the needle members 121a, 121a of the stick portion 121 is executed before the sticking, but it is possible to execute the expansion before the thermal fusion after the sticking and according to circumstances, it is also possible to execute the sticking while expanding.

Depending also on the PFO closing device according to the fourth exemplified embodiment, there are exhibited operations and effects similar to those of the PFO closing device according to the first exemplified embodiment and the expanded needle members 121a, 121a will contact with the foramen ovale valve M2 in a wide region, so that it is possible to sandwich the atrial septum secundum M1 and the foramen ovale valve M2 after the sticking in a wide region and it is possible to make the fusion region of the biological tissue larger. Also, it is possible for the heating of the stick portion 121 to use the electric energy supply means 20 for the thermal fusion simultaneously, so that it is possible to employ the application easily without extending the equipment.

Here, it is also allowed for the lumens L6, L7 of the fourth exemplified embodiment to be formed in shapes expanded in a way of widening toward the distal side similarly as those of the first exemplified embodiment. In addition, it is also allowed for the needle members 121a which constitute the stick portion 121 to be provided by three or more pieces.

### <Fifth Exemplified Embodiment>

FIG. 16 is a plan view showing a stick portion of a PFO closing device according to a fifth exemplified embodiment of the present invention. It should be noted that with respect to the regions having the functions similar to those of the first to the fourth exemplified embodiments, the same reference numerals are used and the explanation thereof is omitted in order to avoid repetition.

The constitution of the PFO closing device according to the fifth exemplified embodiment is similar to the PFO closing device according to the third exemplified embodiment except a stick portion 131.

Clamping means K5 of the fifth exemplified embodiment is constituted, as shown in FIG. 16, by a sandwich member 1 for contacting directly to one side of the atrial septum secundum M1 and a stick portion 131 for being stuck into the foramen ovale valve M2. With respect to the stick portion 131, the distal side thereof is branched into a plurality pieces, and the respective branch portions constitute the needle members 131a, 131a and formed by a shape memory metal. There is used, for example, an Ni-Ti alloy for the shape memory metal. In the use environment temperature (temperature in the body), both the needle members 131a, 131a are in straight shapes as shown by an alternate long and short dash line in FIG. 16 and are housed in the lumen L8 in this state. The operation lever 76b for the needle is operated from this state and the needle members 131a, 131a are protruded and thereafter, by heating the needle members 131a, 131a to a shape-recovery temperature, they are deformed into the stored shapes which are expanded to the distal side.

The heating of the needle members 131a, 131a is executed by supplying electric energy between the stick portion 131 and the sandwich member 1 from the electric energy supply means 20. It should be noted that the amount of supply of the electric energy at that time is less than the energy necessary for fusing the biological tissue and, for example, the shape-recovery temperature is approximately 40°and the temperature for fusing the biological tissue is 70°.

When the PFO closing device according to the present exemplified embodiment is used, the foramen ovale valve M2 is held by the positioning hold means 60 from the left atrium side and thereafter, the lever 76b for the needle is operated and the needle members 131a, 131a are protruded from the catheter 30. Next, owing to the electric energy supply means 20, there is supplied electric energy between the stick portion 131 and the sandwich member, the stick portion 131 is heated until the shape-recovery temperature and the needle members 131a, 131a of the stick portion 131 are expanded. Next, the lever 76b for the needle protrusion is moved forward and the needle members 131a, 131a are stuck at the aimed position of the foramen ovale valve M2. Thereafter, the atrial septum secundum M1 and the foramen ovale valve M2 are held between the sandwich member and the stick portion 131, electric energy is supplied between the stick portion 131 and the sandwich member 1 by the electric energy supply means 20 and the atrial septum secundum M1 and the foramen ovale valve M2 are thermally fused.

After the thermal fusion is ended, the expansion of the needle members 131a, 131a is closed by stopping the supply of the electric energy and lowering the temperature of the needle members 131a, 131a and the expanded needle members 131a, 131a are stored in the catheter 30. Alternatively, it is also possible to close the expansion of the needle members 131a, 131a elastically and to store them by pulling the needle members 131a, 131 of the expanded state into the lumen L8 of the catheter 30.

It should be noted in the present exemplified embodiment that the expansion of the needle members 131a, 131a of the stick portion 131 is executed before the sticking, but it is possible to execute the expansion before the thermal fusion after the sticking and according to circumstances, it is also possible to execute the sticking while expanding.

Depending also on the PFO closing device according to the fifth exemplified embodiment, there are exhibited operations and effects similar to those of the PFO closing device according to the first exemplified embodiment and the expanded needle members 131a, 131a will contact with the foramen ovale valve M2 in a wide region, so that it is possible to sandwich the atrial septum secundum M1 and the foramen ovale valve M2 after the sticking in a wide region and it is possible to make the fusion region of the biological tissue larger. Also, it is possible for the heating of the stick portion 131 to use the electric energy supply means 20 for the thermal fusion simultaneously, so that it is possible to employ the application easily without extending the equipment.

It should be noted that the needle members 131a of the of the stick portion 131 of the fifth exemplified embodiment may be provided in three or more pieces. In addition, the stick portion 131 including the needle members 131a, 131a which are branched into a plurality of pieces may be provided in two or more pieces.

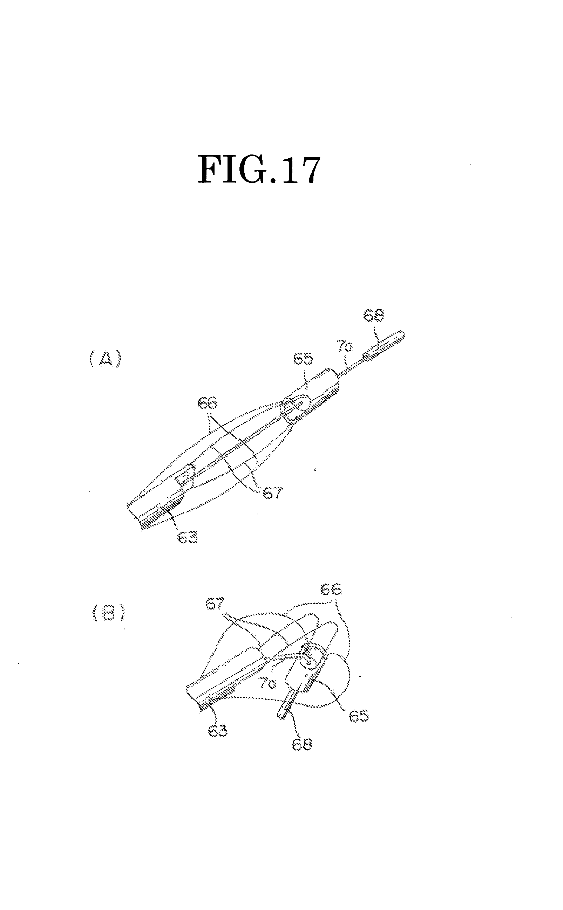

It should be noted that the positioning hold means 60 in the above-mentioned first to fifth exemplified embodiments is not limited by the illustrative embodiments mentioned above. For example, FIG. 17 is an outlined view showing another example of the positioning hold means and, as shown in FIG. 17A, it is also allowed to provide the first elastic wire 66 and the second elastic wire 67 between the distal tip sleeve body 65 and the main tube 63 without providing the middle sleeve body 64 which is provided in the present exemplified embodiment. In this illustrative embodiment, when the main operation rod 7a is moved backward, as shown in FIG. 17B, the second elastic wire 67 is bent and deformed into an arc shape while the first elastic wire 66 is protruded and deformed in an arc shape in the radial direction toward the outside direction. More specifically, it happens that the positioning of the stick portion 2 to the center of the foramen ovale O caused by the first elastic wire 66 and the holding of the foramen ovale valve M2 caused by the bump member 68 and the distal tip sleeve body 65 which are bent by the second elastic wire 67 are to be performed simultaneously based on one action caused by the backward movement of the main operation rod 7a.

The present invention is not limited aforementioned exemplified embodiments and it is possible for a person skilled in the art to employ various modifications within the technical concept of the present invention. In the exemplified embodiment mentioned above, it was explained with respect to a device which is used in a treatment for closing a defect of PFO, but the present invention is not limited to this and the invention is a biological tissue closing device applicable with respect to various kinds of biological tissues and it is possible to use it in case of closing a passway-shaped defect such as a left auricle closing device (Left Atrial Appendage) or in case of thermally necrosing a biological tissue in a predetermined region.

With respect to the PFO closing device of the above-mentioned exemplified embodiment, it is merely housed in the catheter and operates clamping means by the operation member, but it is not limited to this and, for example, it is also possible to carry it until a predetermined position by employing a combination with a so-called catheter having a balloon.

### INDUSTRIAL APPLICABILITY

The present invention can be utilized for a device which can close a defect portion of a PFO simply and also safely.

### BRIEF DESCRIPTION OF DRAWINGS

FIG. 1 is a schematic cross-section view showing a PFO closing device according to a first exemplified embodiment of the present invention;
FIG. 2 is a perspective view of a main portion showing one example of the same device;
FIG. 3 is a cross-section diagram along a 3-3 line in FIG. 2;
FIG. 4A is a diagram equivalent to a cross-section along a 4A-4A line in FIG. 2;
FIG. 4B is a diagram equivalent to a plan view of FIG. 4A;
FIG. 4C is a diagram equivalent to a cross-section along a 4C-4C line in FIG. 4B;
FIG. 5 is a cross-section diagram showing a modified example of a holder;
FIG. 6 is a cross-section diagram showing another modified example of a holder;
FIG. 7 is a cross-sectional outlined view in which a main operation rod is inserted into a foramen ovale;
FIG. 8 is a cross-sectional outlined view of a state in which a foramen ovale valve is held and a needle portion is stuck thereto;
FIG. 9 is a cross-sectional outlined view in which a foramen ovale valve and an atrial septum secundum are sandwiched by a needle portion and a sandwich member;
FIG. 10A to FIG. 10D are outlined views showing operation states of the PFO closing device;
FIG. 11 is a perspective view of a main portion showing a PFO closing device according to a second exemplified embodiment of the present invention;
FIG. 12A is a diagram equivalent to a cross-section along a 12A-12A line in FIG. 11;
FIG. 12B is a diagram equivalent to a plan view of FIG. 12A;
FIG. 12C is a diagram equivalent to a cross-section along a 12C-12C line in FIG. 12B;
FIG. 13 is a perspective view of a main portion showing a PFO closing device according to a third exemplified embodiment of the present invention;
FIG. 14A is a diagram equivalent to a cross-section along a 4A-4A line in FIG. 13;
FIG. 14B is a diagram equivalent to a plan view of FIG. 14A;
FIG. 14C is a diagram equivalent to a cross-section along a 4C-4C line in FIG. 14B;
FIG. 15 is a plan view showing a stick portion of a PFO closing device according to a fourth exemplified embodiment of the present invention;
FIG. 16 is a plan view showing a stick portion of a PFO closing device according to a fifth exemplified embodiment of the present invention; and
FIGS. 17 are outlined views showing another example of positioning hold means, in which FIG. 17A shows a state before deformation and FIG. 17B shows a state after deformation.

### DESCRIPTION OF REFERENCE NUMERALS

1 ··· sandwich member;
2, 101, 111, 121, 131 ··· stick portion;
2a, 101a, 111a, 121a, 131a ··· needle member;
7a ··· main operation rod;
7b, 7c ··· operation member;
20 ··· energy supply means;
30 ··· catheter;
31 ··· guiding catheter;
50 ··· holder;
70 ··· operation unit at hand;
K, K2, K3, K4, K5 clamping means;
L1 to L8 ··· lumen;
M ··· biological tissue;
M1 ··· atrial septum;
M2 ··· foramen ovale valve;
O ··· foramen ovale.

## Claims

1. A device for closing a biological tissue comprising:
clamping means (K) including a stick portion (2) provided at a distal portion of a catheter (30) for sticking to a biological tissue (M1, M2), and a sandwich member (1) for sandwiching said biological tissue (M1, M2) in cooperation with said stick portion (2); and
an electric energy supply means (20) for supplying electric energy to said clamping means (K), in which
said biological tissue (M1, M2) is fused by making said stick portion (2) and said sandwich member (1) of said clamping means (K) be electrode members, by sandwiching said biological tissue (M1, M2) with said clamping means (K) and by supplying electric energy from said electric energy supply means (20), wherein
said clamping means (K) makes each of said stick portion (2) and said sandwich member (1) movable independently with respect to said catheter (30)
wherein
said stick portion (2) is configured with two needle members (2a) and guided by lumens (L1, L2) of said catheter (30)
so as to protrude in a side of said catheter (30); **characterized in that**
said needle members (2a) diverge away from one another in a widening manner in their distal direction; and **in that**
said lumens (L1, L2) are formed so as to tilt toward said side of said catheter.

2. The device for closing a biological tissue according to claim 1, wherein the needle members are mutually expandable and contractible.

3. The device for closing a biological tissue according to any one of claims 1 to 2, wherein said stick portion is made b of a shape memory material, and said needle members expand each other by a shape-recovery temperature higher than the use environment temperature.

4. The device for closing a biological tissue according to claim 3 wherein said stick portion (2) is made by a shape memory material which is heated to the shape-recovery temperature by supplying electric energy from said electric energy supply means to said stick portion (2).

5. The device for closing a biological tissue according to any one of claims 1 to 4, wherein said needle members are deformed before the clamping in a direction apart with respect to said sandwich member.

6. A device for closing a biological tissue according to any one of claims 1 to 5, wherein said catheter (30) is guided by a guiding catheter whose distal portion is bent so as to be easily directed to an opening portion formed at said biological tissue (M1, M2).

7. The device for closing a biological tissue according to any one of claims 1 to 6, wherein said biological tissue (M1, M2) closing device is provided at the proximal portion of said catheter (3 0) with an operation unit at hand for operating said clamping means, and said operation unit at hand and said clamping means (K) are coupled by an operation member inserted into said catheter (30).

## Patentansprüche

1. Vorrichtung zum Verschließen eines biologischen Gewebes, die Folgendes umfasst:
Klemmmittel (K) einschließlich eines Haftanteils (2), der an einem distalen Ende eines Katheters (30) vorgesehen ist zum Haften an ein biologisches Gewebe (M1, M2) sowie ein Sandwichelement (1) zum Zusammenpressen des biologischen Gewebes (M1, M2) im Zusammenspiel mit dem Haftanteil (2); und
ein elektrisches Energieversorgungsmittel (20), um das Klemmmittel (K) mit elektrischer Energie zu versorgen, bei dem
das biologische Gewebe (M1, M2) verschmolzen wird, indem der Haftanteil (2) und das Sandwichelement (1) des Klemmmittels (K) zu Elektrodenträgern gemacht werden, indem das biologische Gewebe (M1, M2) mit dem Klemmmittel (K) zusammengepresst wird und indem elektrische Energie aus dem Energieversorgungsmittel (20) eingespeist wird, wobei
das Klemmmittel (K) sowohl den Haftanteil (2) als auch das Sandwichelement (1) unabhängig voneinander gegenüber dem Katheter (30) beweglich macht
wobei
der Haftanteil (2) mit zwei Nadelelementen (2a) gestaltet ist und durch Lumina (L1, L2) des Katheters (30) geführt wird, so dass er in eine Seite des Katheters (30) ragt,
**dadurch gekennzeichnet, dass**
die Nadelelemente (2a) sich in verbreiternder Weise in deren distaler Richtung voneinander wegbewegen und dadurch, dass
die Lumina (L1, L2) so ausgebildet sind, dass sie gegen die Seite des Katheters geneigt sind.

2. Vorrichtung zum Verschließen eines biologischen Gewebes nach Anspruch 1, wobei die Nadelelemente gegenseitig ausdehnbar und zusammenziehbar sind.

3. Vorrichtung zum Verschließen eines biologischen Gewebes nach einem der Ansprüche 1 bis 2, wobei der Haftanteil aus einem Formgedächtnismaterial hergestellt ist und sich die Nadelelemente gegenseitig ausdehnen durch eine Formrückbildungstemperatur, die höher ist als die Temperatur der Verwendungsumgebung.

4. Vorrichtung zum Verschließen eines biologischen Gewebes nach Anspruch 3, wobei der Haftanteil (2) aus einem Formgedächtnismaterial hergestellt ist, das auf die Formrückbildungstemperatur erhitzt wird durch Einspeisung von elektrischer Energie aus dem Energieversorgungsmittel in das Haftelement (2).

5. Vorrichtung zum Verschließen eines biologischen Gewebes nach einem der Ansprüche 1 bis 4, wobei die Nadelelemente vor dem Anklemmen in getrennter Richtung gegenüber dem Sandwichelement verformt werden.

6. Vorrichtung zum Verschließen eines biologischen Gewebes nach einem der Ansprüche 1 bis 5, wobei der Katheter (30) durch einen Führungskatheter geführt ist, dessen distaler Anteil gebogen ist, so dass er leicht zu einen Öffnungsanteil gerichtet werden kann, der am biologischen Gewebe (M1, M2) gebildet wird.

7. Vorrichtung zum Verschließen eines biologischen Gewebes nach einem der Ansprüche 1 bis 6, wobei die Vorrichtung zum Verschließen von biologischem Gewebe (M1, M2) am proximalen Anteil des Katheters (30) mit einer Betriebseinheit versehen ist, die zum Betreiben des Klemmmittels griffbereit ist und die griffbereite Betriebseinheit und das Klemmmittel (K) über ein Betriebselement gekoppelt sind, das in den Katheter (30) eingefügt ist.

## Revendications

1. Dispositif pour fermer un tissu biologique comprenant :
un moyen de pincement (K) comprenant une partie bâton (2) placée au niveau d'une partie distale d'un cathéter (30) et destinée à être collée à un tissu biologique (M1, M2), et un élément de sandwich (1) pour prendre en sandwich ledit tissu biologique (M1, M2) en coopération avec ladite partie bâton (2) ; et
un moyen d'alimentation en énergie électrique (20) pour fournir de l'énergie électrique audit moyen de pincement (K), dans lequel
ledit tissu biologique (M1, M2) est fondu en faisant de ladite partie bâton (2) et dudit élément de sandwich (1) du moyen de pincement (K) des éléments d'électrodes, en prenant en sandwich ledit tissu biologique (M1, M2) avec ledit moyen de pincement (K) et en fournissant de l'énergie électrique à partir dudit moyen d'alimentation en énergie électrique (20), dans lequel
ledit moyen de pincement (K) rend chacun desdits partie bâton (2) et élément de sandwich (1) mobile indépendamment par rapport audit cathéter (30),
dans lequel ladite partie bâton (2) est configurée avec deux éléments d'aiguille (2a) et guidée par des lumières (L1, L2) du cathéter (30) afin de faire saillie dans un côté dudit cathéter (30) ;
**caractérisé en ce que** lesdits éléments d'aiguille (2a) divergent l'un par rapport à l'autre de manière élargie dans leur direction distale ;
et **en ce que** lesdites lumières (L1, L2) sont formées de façon à être inclinées vers ledit côté du cathéter.

2. Dispositif pour fermer un tissu biologique selon la revendication 1, dans lequel les éléments d'aiguille peuvent être mutuellement étendus et contractés.

3. Dispositif pour fermer un tissu biologique selon l'une quelconque des revendications 1 à 2, dans lequel ladite partie bâton est faite d'un matériau à mémoire de forme, et lesdits éléments d'aiguille s'étendent mutuellement au moyen d'une température de récupération de forme supérieure à la température ambiante d'utilisation.

4. Dispositif pour fermer un tissu biologique selon la revendication 3, dans lequel ladite partie bâton (2) est faite par un matériau à mémoire de forme que l'on chauffe jusqu'à la température de récupération de forme en fournissant de l'énergie électrique provenant dudit moyen d'alimentation en énergie électrique à ladite partie bâton (2).

5. Dispositif pour fermer un tissu biologique selon l'une quelconque des revendications 1 à 4, dans lequel lesdits éléments d'aiguille sont déformés avant le pincement dans une direction qui s'écarte dudit élément de sandwich.

6. Dispositif pour fermer un tissu biologique selon l'une quelconque des revendications 1 à 5, dans lequel ledit cathéter (30) est guidé par un cathéter de guidage dont la partie distale est courbée afin de pouvoir être aisément dirigée dans une partie d'ouverture formée au niveau dudit tissu biologique (M1, M2).

7. Dispositif pour fermer un tissu biologique selon l'une quelconque des revendications 1 à 6, dans lequel ledit dispositif de fermeture d'un tissu biologique (M1, M2) est pourvu dans la partie proximale dudit cathéter (30) d'une unité d'actionnement à main pour actionner ledit moyen de pincement, et ladite unité d'actionnement à main et ledit moyen de pincement (K) sont accouplés par un élément d'actionnement inséré dans ledit cathéter (30).
